# EUROPEAN PATENT SPECIFICATION

(11) **EP 1 865 891 B1**
(45) Date of publication and mention of the grant of the patent: **03.05.2017**
(21) Application number: 06739304.1
(22) Date of filing: 22.03.2006
(51) Int. Cl.: A61F 2/44

(54) **MINIMALLY INVASIVE SPINE RESTORATION DEVICES**
MINIMAL INVASIVE WIRBELSÄULENWIEDERHERSTELLUNGSVORRICHTUNGEN
DISPOSITIFS DE RESTAURATION DE COLONNE VERTEBRALE MINIMALEMENT INVASIVE

(30) Priority: 22.03.2005 US 664441 P; 22.09.2005 US 719427 P; 20.12.2005 US 752277 P
(43) Date of publication of application: 19.12.2007
(73) Proprietor: Gmedelaware 2 LLC, Wilmington DE 19801 (US)
(72) Inventor: KUIPER, Mark, K., Seattle, WA 98118 (US); JONES, Lawrence, R., Conifer, CO 80433 (US); FINAZZO, Anthony, V., Lake Forest Park, Washington 98155 (US); SUH, Sean, Sung-Ho, Kirkland, WA 98034 (US); OHRT, John, Arthur, Redmond, WA 98052 (US); BROMAN, Richard, J., Kirkland, WA 98034 (US); ABIDIN, Cin, Issaquah, WA 98029 (US); FUNK, Michael, J., North Bend, Washington 98045 (US); YUAN, Hansen, Fayetteville, NY 13066 (US); BERG, Phillip, Federal Way, Washington 98003 (US); REILEY, Mark, A., Piedmont, CA 94611 (US); STONE, Martha, K., Lake Forrest Park, 98155 (US); STINSON, David, Woodinville, WA 98072 (US); QUEST, Matthew, Bothell, WA 98011 (US)
(74) Representative: Potter Clarkson LLP
(86) International application number: PCT/US2006/010454
(87) International publication number: WO 2006/102443

(56) References cited:
- WO-A2-94/06361
- US-A1- 2004 127 906
- US-A1- 2004 230 304
- US-A1- 2005 049 705

## Description

### CROSS-REFERENCE

This application claims the benefit of U.S. Provisional Application No. 60/664,441, to Michael J. Funk et al, filed March 22, 2005, and entitled "Minimally Invasive Facet Replacement"; U.S. Provisional Application No. 60/719,427, to Michael J. Funk et al., filed September 22, 2005, entitled "Prosthesis, Tools and Methods for Replacement of Natural Facet Joints with Artificial Facet Joint Surfaces"; and U.S. Provisional Application 60/752,277 to Christopher Ralph et al., filed December 20,2005, entitled "Spinal Joint Replacement Systems".

### FIELD OF THE INVENTION

The present invention generally relates to devices and surgical methods for the treatment of various types of pathologies of the spine. More specifically, the present invention is directed to several different types of minimally invasive devices, methods, systems and kits for treating injured or diseased facet joints, intervertebral joints and adjacent anatomy of the spine.

### BACKGROUND OF THE INVENTION

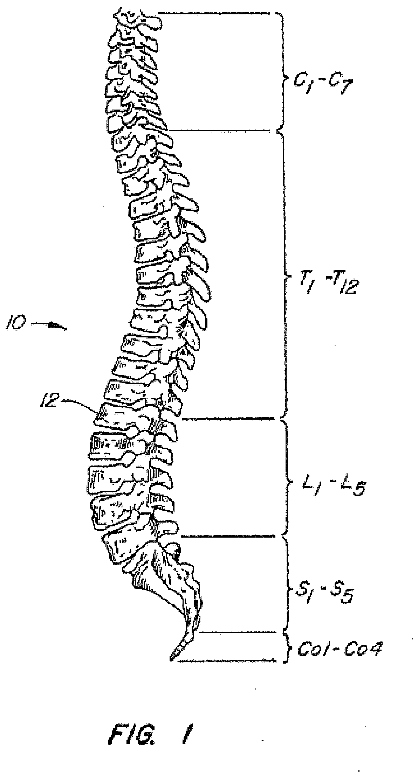

Back pain, particularly in the "small of the back" or lumbosacral (L4-S1) region, shown in **FIG.1****,** is a common ailment. In many cases, the pain severely limits a person's functional ability and quality of life. Such pain can result from a variety of spinal pathologies. Through disease or injury, the laminae, spinous process, articular processes, or facets of one or more vertebral bodies can become damaged, such that the vertebrae no longer articulate or properly align with each other. This can result in an undesired anatomy, loss of mobility, and pain or discomfort.

In many cases, the vertebral facet joints can be damaged by either traumatic injury or by various disease processes. These disease processes include osteoarthritis, ankylosing spondylolysis, and degenerative spondylolisthesis. Moreover, the facet joint has been implicated as a potential cause of neck pain for persons having whiplash. Aside from pain coming from the facets themselves, such damage to the facet joints can often result in eventual degeneration, abrasion, or wearing down of the facet joints, eventually resulting in pressure on nerves, also called "pinched" nerves, or nerve compression or impingement. The result is further pain, misaligned anatomy, and a corresponding loss of mobility. Pressure on nerves can also occur without an anatomic or functional manifestation of a disease, or pathology, at the facet joint, *e.g*., as a result of a herniated disc.

Many spinal pathologies mandating repair and/or replacement of an intervertebral disc (including many of those that may be currently treated through spinal fusion, interspinous distraction and/or dynamic stabilization), can often be traced back to degeneration, disease and/or failure of the facet joints. Alteration of the facet joint biomechanics resulting from an anatomic or functional manifestation of a disease can adversely affect the loading and biomechanics of the intervertebral disc, eventually resulting in degeneration, damage and/or failure of the intervertebral disc.

One type of conventional treatment of facet joint pathology is spinal stabilization, also known as intervertebral stabilization. Intervertebral stabilization desirably prevents relative motion between vertebrae of the spine. By preventing movement, pain can be reduced. Stabilization can be accomplished by various methods. One method of stabilization is spinal fusion. Another method of stabilization is fixation of any number of vertebrae to stabilize and prevent movement of the vertebrae. In addition, where compression or subsidence of the disc and/or facet joints has occurred, the physician can utilize fusion devices such as pedicle screw and rods systems, or interbody fusion cages, to elevate or "jack up" the compressed level, desirably obtaining a more normal anatomical spacing between the vertebral bodies.

Various devices are known for fixing the spine and/or sacral bone adjacent the vertebra, as well as attaching devices used for fixation, are known in the art, including: U.S. Patent Nos. 6,290,703, to Ganem, for Device for Fixing the Sacral Bone to Adjacent Vertebrae During Osteosynthesis of the Backbone; 6,547,790, to Harkey, III, et al., for Orthopaedic Rod/Plate Locking Mechanisms and Surgical Methods; 6,074,391, to Metz-Stavenhagen, et al., for Receiving Part for a Retaining Component of a Vertebral Column Implant; 5,569,247, to Morrison, for Enhanced Variable Angle Bone Bolt; 5,891,145, to Morrison, et al., for Multi-Axial Screw; 6,090,111, to Nichols, for Device for Securing Spinal Rods; 6,451,021, to Ralph, et al., for Polyaxial Pedicle Screw Having a Rotating Locking Element; 5,683,392, to Richelsoph, et al., for Multi-Planar Locking Mechanism for Bone Fixation; 5,863,293, to Richelsoph, for Spinal Implant Fixation Assembly; 5,964,760, to Richelsoph, for Spinal Implant Fixation Assembly; 6,010,503, to Richelsoph, et al., for Locking Mechanism; 6,019,759, to Rogozinski, for Multi-Directional Fasteners or Attachment Devices for Spinal Implant Elements; 6,540,749, to Schafer, et al., for Bone Screw; 6,077,262, to Schlapfer, for Posterior Spinal Implant; 6,248,105, to Schlapfer, et al., for Device for Connecting a Longitudinal Support with a Pedicle Screw; 6,524,315, to Selvitelli, et al., for Orthopaedic Rod/Plate Locking Mechanism; 5,797,911, to Sherman, et al., for Multi-Axial Bone Screw Assembly; 5,879,350, to Sherman, et al., for Multi-Axial Bone Screw Assembly; 5,885,285, to Simonson, For Spinal Implant Connection Assembly; 5,643,263, to Simonson for Spinal Implant Connection Assembly; 6,565,565, to Yuan, et al., for Device for Securing Spinal Rods; 5,725,527, to Biederman, et al., for Anchoring Member; 6,471,705, to Biederman, et al., for Bone Screw; 5,575,792, to Errico, et al., for Extending Hook and Polyaxial Coupling Element Device for Use with Top Loading Rod Fixation Devices; 5,688,274, to Errico, et al., for Spinal Implant Device having a Single Central Rod and Claw Hooks; 5,690,630, to Errico, et al., for Polyaxial Pedicle Screw; 6,022,350, to Ganem, for Bone Fixing Device, in Particular for Fixing to the Sacrum during Osteosynthesis of the Backbone; 4,805,602, to Puno, et al., for Transpedicular Screw and Rod System; 5,474,555, to Puno, et al., for Spinal Implant System; 4,611,581, to Steffee, for Apparatus for Straightening Spinal Columns; 5,129,900, to Asher, et al., for Spinal Column Retaining Method and Apparatus; 5,741,255, to Krag, et al., for Spinal Column Retaining Apparatus; 6,132,430, to Wagner, for Spinal Fixation System; U.S. Publication No. 2002/0120272, and to Yuan, et al., for Device for Securing Spinal Rods.

Another type of conventional spinal treatment is decompressive laminectomy. Where spinal stenosis (or other spinal pathology) results in a narrowing of the spinal canal and/or the intervertebral foramen (through which the spinal nerves exit the spine), and neural impingement, compression and/or pain results, the tissue(s) (hard and/or soft tissues) causing the narrowing may need to be resected and/or removed. A procedure which involves excision of part or all of the laminae and other tissues to relieve compression of nerves is called a decompressive laminectomy. *See,* for example, U.S. Patent Nos. 5,019,081, to Watanabe, for Laminectomy Surgical Process; 5,000,165, to Watanabe, for Lumbar Spine Rod Fixation System; and 4,210,317, to Spann, et al., for Apparatus for Supporting and Positioning the Arm and Shoulder. Depending upon the extent of the decompression, the removal of support structures such as the facet joints and/or connective tissues (either because these tissues are connected to removed structures or are resected to access the surgical site) may result in instability of the spine, necessitating some form of supplemental support such as spinal fusion, discussed above.

US 2004/0230304 discloses a prosthesis for replacing a natural facet joint with artificial facet joint surfaces. The prosthesis comprises caudad and cephalad facet joint elements that are fixed to vertebrae with screws.

### SUMMARY OF THE INVENTION

The present invention provides a facet joint restoration device according to claim 1.

While spinal fusion has become the "gold standard" for treating many spinal pathologies, including pathologies such as neurological involvement, intractable pain, instability of the spine and/or disc degeneration, it would be desirable to reduce and/or obviate the need for spinal fusion procedures by providing devices and systems that stabilize, or preserve motion of the spinal motion segment (including, but not limited to, facet joint repair or replacement, intervertebral disk replacement or nucleus replacement, implantation of interspinous spacers and/or dynamic stabilization devices, and/or facet injections).

The present invention includes the recognition that many spinal pathologies eventually requiring surgical intervention can be traced back, in their earlier stage(s), to some manner of a degeneration, disease and/or failure of the facet joints. Moreover, spinal fusion procedures can eventually require further surgical intervention. For example, degeneration of facet joints can result in an unnatural loading of an intervertebral disc, eventually resulting in damage to the disc, including annular bulges and/or tears. Similarly, degeneration and/or failure of a facet joint can potentially lead to slipping of the vertebral bodies relative to one another, potentially resulting in spondylolisthesis and/or compression of nerve fibers. In addition, degeneration of the facet joints themselves can become extremely painful, leading to additional interventional procedures such as facet injections, nerve blocks, facet removal, facet replacement, and/or spinal fusion. Thus, if the degenerating facet joint can be treated at an early stage, the need for additional, more intrusive procedures, may be obviated and damage that has already occurred to spinal structures such as the intervertebral disc of the treated level (as well as the disc and/or facets of other spinal levels) may be slowed, halted or even reversed.

Further, the invention includes the ability to accommodate anatomical variability to treat all vertebral levels, including L3-L4, L4-L5 and L5-S1, across a majority of the patient population.

The various embodiments disclosed and discussed herein may be utilized to restore and/or maintain varying levels of the quality or state of motion or mobility and/or motion preservation in the treated vertebral bodies. Depending upon the extent of facet joint degradation, and the chosen treatment regime(s), it may be possible to completely restore the quality or state of motion across the entire spinal motion segment, across one or more of the facet joints, or restore limited motion across the facet joint(s) to reduce or obviate the need for further treatment of the spinal motion segment.

In some embodiments, the flexible member is adapted to engage a lamina of the first vertebrae. In other embodiments, the cephalad facet joint element further comprises a plate with an anchoring mechanism adapted to engage a lamina of the first vertebrae. The anchoring mechanisms can be any suitable mechanism, including, for example, one or more anchoring mechanisms selected from the group consisting of teeth, ridges, nubs, serrations, granulations, a stem, a screw and spikes. In some embodiments, the cephalad facet joint element can be further adapted to comprise a second anchoring mechanism for securing the cephalad facet joint element to the first vertebrae. Further, the connector can be adapted to provide for fixation to a pedicle of the second vertebrae. In some embodiments it may be desirable for the second anchoring mechanism to further comprise a bony in-growth surface. As will be appreciated by those skilled in the art, in still other embodiments, the device can be configured to replace tissue removed from the facet joint, such as where the facet joint is resected. In still other embodiments, the device is adapted to restore or maintain motion or mobility for the facet joint. Further, a surface of one of the cephalad facet joint element or caudad facet joint element can be adapted to contour to an opposing mating surface. For example, the artificial caudad joint is a caudad cup having a concave surface. In some embodiments, the flexible member is a flexible cable. The flexible cable may be surrounded by a tube and/or may be further adapted to engage a lock. A spring washer adapted to engage a surface of the first or second vertebrae can be used in some embodiments, if desired. Further, it may be desirable to employ a malleable plate adapted to engage a laminar surface to support the cephalad facet joint element during implantation in other embodiments.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIG. 1 is a lateral elevation view of a normal human spinal column;
FIG. 2A is a superior view of a normal human lumbar vertebra;
FIG. 2B is a lateral elevational view of two vertebral bodies forming a functional spinal unit;
FIG. 2C is a posterior view of two vertebral bodies forming a functional spine unit and illustrating a coronal plane across a facet joint;
FIG. 2D is a cross-sectional view of a single facet joint in a spinal column taken along a coronal plane;
FIG. 2E is a posterolateral oblique view of a vertebrae from a human spinal column;

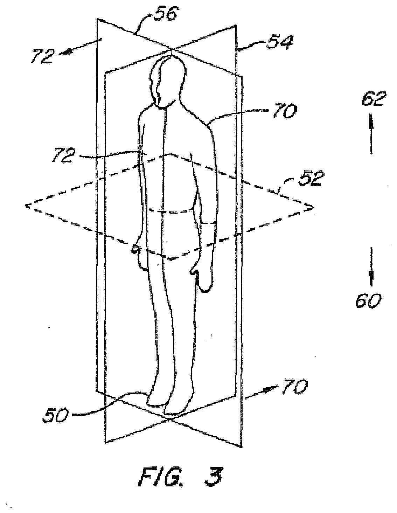

FIG. 3 is a perspective view of the anatomical planes of the human body;
FIG.4 depicts an embodiment of a facet replacement device according to the invention;
FIG.5 illustrates a bilateral facet replacement system according to the invention;
FIG.6A illustrates two components of the facet replacement system;
FIGS.6B-C illustrate the two components illustrated in FIG. 6A in combination from different perspectives;
FIGS.7A-C illustrate an implanted facet replacement device according to the invention from a posterior and lateral perspective;
FIGS. 8A-D illustrate an implanted facet replacement device according to another embodiment of the invention from a posterior and lateral perspective;
FIGS.9 A-B illustrate a facet replacement device according to another embodiment of the invention from a side view and a top view;

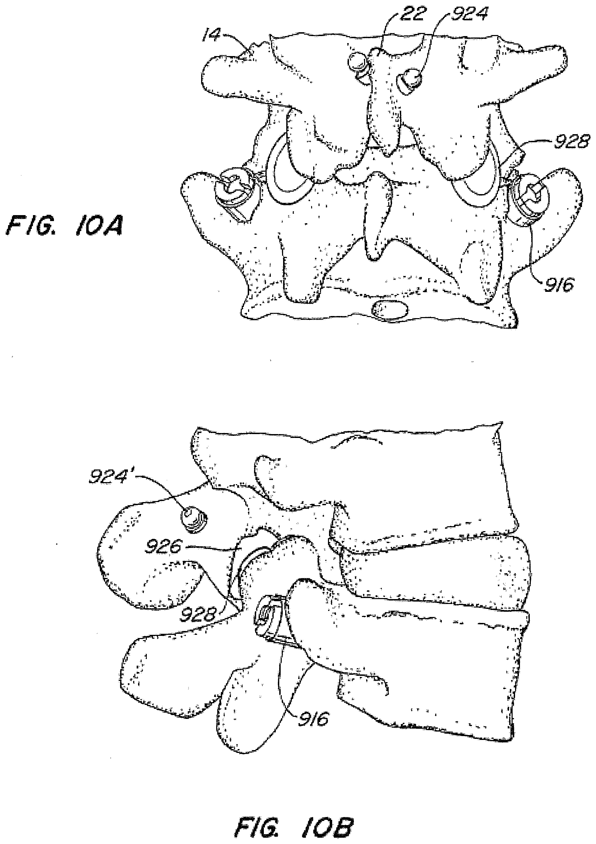

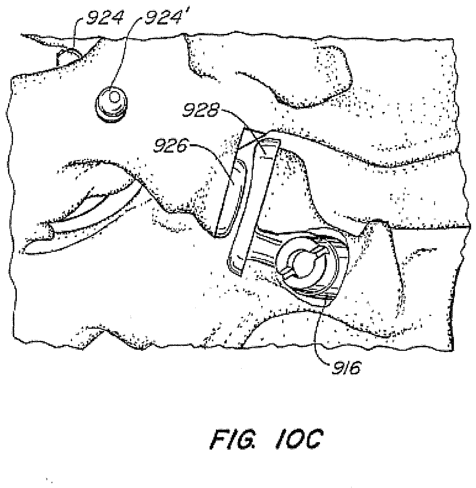

FIGS. 10A-C illustrate the facet replacement device of FIGS.9A-B implanted from a posterior and lateral view;
FIG. 11 illustrates a bilateral facet replacement system with a cross-bar;
FIGS. 12A-B illustrate a bilateral facet replacement system with a cross-bar according to another embodiment of the invention;
FIGS. 12C-D illustrates the facet replacement system implanted from different perspectives;
FIGS.13A and C illustrate cross-sections of an alternate cephalad bearing fixation system.

### DETAILED DESCRIPTION OF THE INVENTION

The invention relates generally to implantable devices, apparatus or mechanisms that are suitable for implantation within a human body to restore, augment, and/or replace hard tissue, soft tissue and/or connective tissue, including bone and cartilage, and systems for treating the anatomic or functional manifestation of injury or diseases, such as spinal pathologies. In some instances, the implantable devices can include devices designed to replace missing, removed, or resected body parts or structure. The implantable devices, apparatus or mechanisms are configured such that the devices can be formed from parts, elements or components which alone or in combination comprise the device. The implantable devices can also be configured such that one or more elements or components are formed integrally to achieve a desired physiological, operational or functional result such that the components complete the device. Functional results can include the surgical restoration and functional power of a joint, controlling, limiting or altering the functional power of a joint, and/or eliminating the functional power of a joint by preventing joint motion. Portions of the device can be configured to replace or augment existing anatomy and/or implanted devices, and/or be used in combination with resection or removal of existing anatomical structure.

The devices of the invention are designed to interact with the human spinal column 10, as shown in FIG. 1, which is comprised of a series of thirty-three stacked vertebrae 12 divided into five regions. The cervical region includes seven vertebrae, known as C1-C7. The thoracic region includes twelve vertebrae, known as T1-T12. The lumbar region contains five vertebrae, known as L1-L5. The sacral region is comprised of five normally-fused vertebrae, known as S1-S5, while the coccygeal region contains four fused vertebrae, known as Co1-Co4.

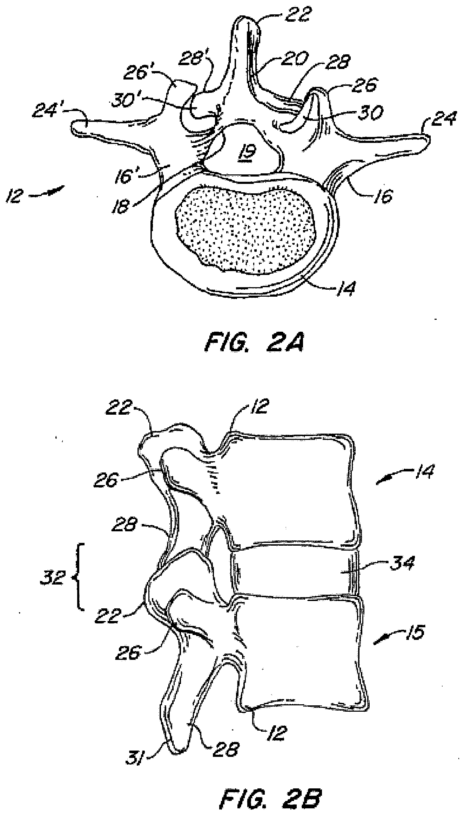

An example of one vertebra is illustrated in FIG. 2A which depicts a superior plan view of a normal human
lumbar vertebra ***12.*** Although human lumbar vertebrae vary somewhat according to location, the vertebrae share many common features. Each vertebra ***12*** includes a vertebral body ***14.*** Two short boney protrusions, the pedicles ***16,16',*** extend dorsally from each side of the vertebral body ***14*** to form a vertebral arch ***18*** which defines the vertebral foramen ***19***.

At the posterior end of each pedicle ***16,*** the vertebral arch ***18*** flares out into broad plates of bone known as the laminae ***20.*** The laminae ***20*** fuse with each other to form a spinous process ***22.*** The spinous process ***22*** provides for muscle and ligamentous attachment. A smooth transition from the pedicles ***16*** to the laminae ***20*** is interrupted by the formation of a series of processes.

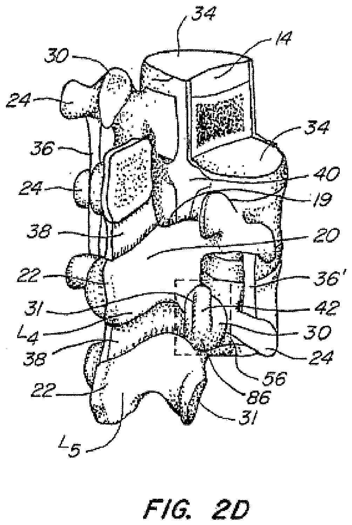

Two transverse processes ***24, 24'*** thrust out laterally, one on each side, from the junction of the pedicle ***16*** with the lamina ***20.*** The transverse processes ***24, 24'*** serve as levers for the attachment of muscles to the vertebrae ***12.*** Four articular processes, two superior ***26, 26*'** and two inferior ***28, 28'**,* also rise from the junctions of the pedicles ***16*** and the laminae ***20.*** The superior articular processes ***26, 26'*** are sharp oval plates of bone rising upward on each side of the vertebrae, while the inferior processes ***28, 28'*** are oval plates of bone that jut downward on each side. *See also* **FIGS. 2B** and **2D**.

The superior and inferior articular processes ***26*** and ***28*** each have a natural bony structure known as a facet. The superior articular facet ***30*** faces medially upward, while the inferior articular facet ***31*** (see **FIGS. 2B-E**) faces laterally downward. When adjacent vertebrae ***12*** are aligned, the facets ***30*** and ***31,*** capped with a smooth articular cartilage and encapsulated by ligaments, interlock to form a facet joint ***32***. The facet joints are apophyseal joints that have a loose capsule and a synovial lining.

As discussed above, the facet joint ***32*** is composed of a superior facet and an inferior facet. The superior facet is formed by the vertebral level below the joint ***32,*** and the inferior facet is formed in the vertebral level above the joint 32. For example, in the **L4-L5** facet joint shown in **FIG**.**2B****,** the superior facet of the joint ***32*** is formed by bony structure on the ***L5*** vertebra (*i.e.*, a superior articular surface and supporting bone **26** on the **L5** vertebra), and the inferior facet of the joint ***32*** is formed by bony structure on the **L4** vertebra (*i.e.*, an inferior articular surface and supporting bone ***28*** on the **L4** vertebra). The angle formed by a facet joint located between a superior facet and an inferior facet changes with respect to the midline of the spine depending upon the location of the vertebral body along the spine ***10*** (**FIG. 1**). The facet joints do not, in and of themselves, substantially support axial loads unless the spine is in an extension posture (lordosis). As would be appreciated by those of skill in the art, the orientation of the facet joint for a particular pair of vertebral bodies changes significantly from the thoracic to the lumbar spine to accommodate a joint's ability to resist flexion-extension, lateral bending, and rotation.

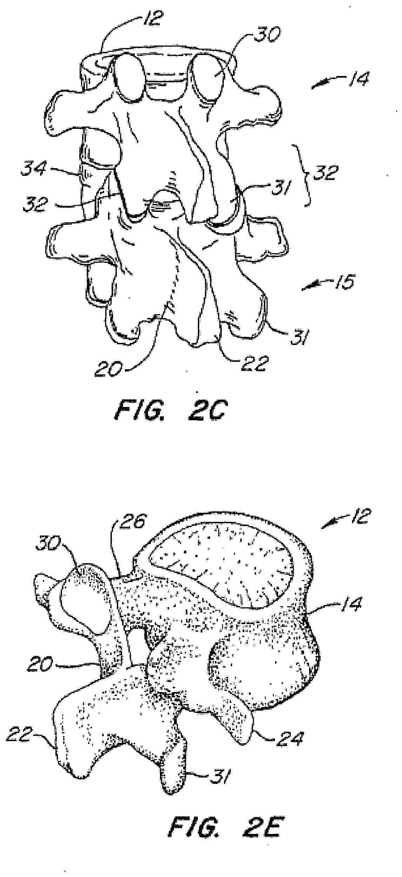

An intervertebral disc ***34*** between each adjacent vertebra ***12*** (with stacked vertebral bodies shown as ***14*, *15*** in **FIGS**. **2B****, C**, **E**) permits gliding movement between the vertebrae ***12.*** The structure and alignment of the vertebrae ***12*** thus permit a range of movement of the vertebrae ***12*** relative to each other. **FIG. 2E** illustrates a posterolateral oblique view of a vertebrae ***12,*** further illustrating the curved surface of the superior articular facet ***30*** and the protruding structure of the inferior facet ***31*** adapted to mate with the opposing superior articular facet. As discussed above, the position of the inferior facet ***31*** and superior facet ***30*** varies on a particular vertebral body to achieve the desired biomechanical behavior of a region of the spine.

Thus, the overall spine comprises a series of functional spinal units that are a motion segment consisting of two adjacent vertebral bodies (*e.g., **14, 15*** of **FIGS**. **2B**, **C**, **E**), the intervertebral disc (*e.g., **34*** of **FIGS. 2B****, C, E**), associated ligaments, and facet joints (*e.g., **32*** of **FIG. 2D**). *See,* Posner. I, et al. A biomechanical analysis of the clinical stability of the lumbar and lumbrosacral spine. Spine 7:374-389 (1982).

As previously described, a natural facet joint, such as facet joint ***32*** (**FIGS**. **2B-D**), has a superior facet ***30*** and an inferior facet ***31*** (shown in **FIG**. **2B**, **C**, **E**). In anatomical terms, the superior facet of the joint is formed by the vertebral level below the joint, which can thus be called the "caudad" portion of the facet joint because it is anatomically closer to the tail bone or feet of the person. The inferior facet of the facet joint is formed by the vertebral level above the joint, which can be called the "cephalad" portion of the facet joint because it is anatomically closer to the head of the person. Thus, a device that, in use, replaces the caudad portion of a natural facet joint (*i.e*., the superior facet ***30***) can be referred to as a "caudad" device. Likewise, a device that, in use, replaces the cephalad portion of a natural facet joint (*i.e.,* the inferior facet ***31***) can be referred to a "cephalad" device.

As will be appreciated by those skilled in the art, it can be difficult for a surgeon to determine the precise size and/or shape necessary for an implantable device until the surgical site has actually been prepared for receiving the device. In such case, the surgeon typically would desire to quickly deploy a family of devices and/or device components possessing differing sizes and/or shapes during the surgery. Thus, embodiments of the spinal devices of the present invention include modular designs that are either or both configurable and adaptable. Additionally, the various embodiments disclosed herein may also be formed into a "kit" or system of modular components that can be assembled *in situ* to create a patient specific solution. As will be appreciated by those of skill in the art, as imaging technology improves, and mechanisms for interpreting the images (*e.g*., software tools) improve, patient specific designs employing these concepts may be configured or manufactured prior to the surgery. Thus, it is within the scope of the invention to provide for patient specific devices with integrally formed components that are pre-configured.

The devices of the present invention are configurable such that the resulting implantable device is selected and positioned to conform to a specific anatomy or desired surgical outcome. The adaptable aspects of embodiments of the present invention provide the surgeon with customization options during the implantation or revision procedure. It is the adaptability of the present devices and systems that also provides adjustment of the components during the implantation procedure to ensure optimal conformity to the desired anatomical orientation or surgical outcome. An adaptable modular device of the present invention allows for the adjustment of various component-to-component relationships. One example of a component-to-component relationship is the rotational angular relationship between an anchoring device and the device to be anchored. Other examples of the adaptability of modular device of the present invention are as described in greater detail below. Configurability may be thought of as the selection of a particular size of component that together with other component size selections results in a "custom fit" implantable device. Adaptability then can refer to the implantation and adjustment of the individual components within a range of positions in such a way as to fine tune the "custom fit" devices for an individual patient. The net result is that embodiments of the modular, configurable, adaptable spinal device and systems of the present invention allow the surgeon to alter the size, orientation, and relationship between the various components of the device to fit the particular needs of a patient during the actual surgical procedure.

To prepare the anatomy for implantation of the devices and systems disclosed herein, it may be desirable to alter or remove anatomy from the patient. For example, common ligaments, such as capsular ligaments, anterior longitudinal ligaments, interspinous ligaments and/or ligamentum flavum may be altered or removed, as well as portions of the cephalad and/or caudad vertebra, including inferior/superior facets, or portions thereof. Alternatively, less-invasive and/or minimally-invasive surgical tools and techniques are provided that, among other things, limit the need for resection and/or alteration of such anatomy, which desirably allows for greater retention of natural anatomical features that can (1) stabilize the spine, thereby desirably reducing loads experienced by the facet replacement device, and/or (2) load-share with the facet joint replacement device in bearing physiological loads.

In order to understand the configurability, adaptability and operational aspects of the invention, it is helpful to understand the anatomical references of the body ***50*** with respect to which the position and operation of the devices, and components thereof, are described. There are three anatomical planes generally used in anatomy to describe the human body and structure within the human body: the axial plane ***52*,** the sagittal plane ***54*** and the coronal plane ***56*** (see **FIG**. **3****).** Additionally, devices and the operation of devices are better understood with respect to the caudad ***60*** direction and/or the cephalad direction ***62*.** Devices positioned within the body can be positioned dorsally ***70*** (or posteriorly) such that the placement or operation of the device is toward the back or rear of the body. Alternatively, devices can be positioned ventrally ***72*** (or anteriorly) such that the placement or operation of the device is toward the front of the body. Various embodiments of the spinal devices and systems of the present invention may be configurable and variable with respect to a single anatomical plane or with respect to two or more anatomical planes. For example, a component may be described as lying within and having adaptability in relation to a single plane. For example, an anchoring device may be positioned in a desired location relative to an axial plane and may be moveable between a number of adaptable positions or within a range of positions. Similarly, the various components can incorporate differing sizes and/or shapes in order to accommodate differing patient sizes and/or anticipated loads.

Turning back to **FIG**. **2D**, a vertebral body ***14*** is depicted in at least partial cross-section along, for example a sagittal plane ***54*** and a facet joint ***32*** is depicted in a coronal plane ***56***, As will be appreciated, the orientation of a facet joint ***32*** in any plane of the body changes depending upon the location of a particular joint within the spinal column, this example is provided for illustration purposes only.

The facet joint ***32,*** is formed from a superior articular facet ***30*** and an inferior articular facet ***31*.** The inferior articular facet ***31*** has a cephalad facet surface and the superior articular facet ***30*** has a caudad facet surface. When healthy and normal, each of these surfaces has an articulating cartilage layer positioned adjacent the facet surfaces to improve the movement of the facet joint ***32*** in operation. In addition to the caudad facet surface and the cephalad facet surface that comprise the opposing joint surfaces, each of the superior articular facet ***30*** and the inferior articular facet ***31*** may have additional surfaces on the sides of the facets. A facet capsule ***86*** is also provided that surrounds the facet joint ***32*** and to communicate with the various surfaces on the sides of the superior articular facet ***30*** and the inferior articular facet ***31*.** Where the anatomic or functional manifestations of a disease has resulted in a spinal pathology, facet joint degradation can occur, which includes wear of the articulating surface of the facet joint. Normally, the peripheral, cortical rim of the joint is not affected, or is minimally affected. With hypertrophic facets, the mass of cortical bone and action of the osteophytes can make the facet larger than normal as the facet degenerates. When a facet begins to wear, the biomechanics of the functional spine unit are altered, which can cause further damage to the facet joint as well as pain. Moreover, such alteration of the biomechanics can compromise the integrity of the remainder of the functional spinal unit, and lead to intervertebral disc degredation and damage, further facet joint degredation and damage, spondylolithesis and/or reductions/changes in disc height, as well as the potential occurrence of spinal stenosis (all of which could occur not only in the affected spinal level, but in other spinal levels as well).

**FIG**. **4** depicts an embodiment of facet replacement device according to the invention. The device is an artificial facet joint replacement device ***410*** configured to replace a portion of a natural facet joint. The device 410 comprises fixation elements ***412*** and ***422*** that connect the device ***410*** to the corresponding vertebral structures supporting the caudad and cephalad components of the natural facet joint. In this embodiment, the caudad component of the device ***410*** incorporates a screw or stem as the fixation element ***412*** that connects into or near the pedicle of the caudad vertebral body. An adjustable connection ***414*** connects the base ***416*** of the fixation element ***412*** to the artificial caudad joint ***428,*** allowing adjustment and/or rotation of the artificial caudad joint ***428*** along and/or around one or more axes relative to the fixation element ***412*.** Desirably, the construction is adapted and configured to permit continuous adjustment through relative rotation of the facet joint element and the fixation element around and/or along many different axes through an adjustability range, up to a motion limit provided by a limit stop (if any). In other embodiments, however, the number of axes of rotation may be limited, and the movement may be permitted only in discrete increments. In various embodiments the facet joint element may be moved medially, laterally, superiorly and/or inferiorly with respect to the fixation element. The cephalad fixation element ***422*** comprises a cable which can be securable through, for example, the lamina or spinous process **(****FIGs. 2A-D****, *22*)** by use of an anchor ***424*.** Desirably, the use of a flexible cable allows for varying alignment of the cable relative to the artificial cephalad joint ***426*.** For example, the surgeon may wish to create an opening through the lamina and/or spinous process which is optimal for fixation strength of the anchor, but which does not extend along a pre-determined longitudinal axis of the artificial cephalad joint ***426*.** In such a case, the actual orientation of the opening relative to a desired position of the artificial cephalad joint could of infinite variability, which can easily be accommodated by the flexible cable. Desirably, the cable will draw the artificial cephalad joint into intimate contact with the outer surface of the cephalad vertebral body, securing the joint to the vertebral body and rendering the joint capable of immediately bearing load (if desired) as well as facilitating fixation and/or bony ingrowth into the joint. To properly orient the artificial cephalad joint, the surface of the cephalad vertebral body may be pre-shaped such that, when the artificial cephalad joint is in intimate contact with the targeted vertebral surface, it mates with the pre-shaped surface and thus occupies a desired position and/or orientation. Alternatively, a series of differently sized and/or shaped artificial cephalad joints could be provided. A cephalad bearing ***426*** is connected to the artificial cephalad joint, the bearing ***426*** having a surface ***427*** which is adapted to interact with an opposing surface of the artificial caudad joint ***428*.** If desired, a series of cephalad bearings of differing shapes, sizes and/or orientations and/or lengths can be provided to accommodate different objectives, including alteration of bearing height/orientation relative to the fixation element, to accommodate different loading conditions due to other surgical treatments (i.e., artificial disc replace of the same or other spinal level, annular repair, nucleus replacement, dynamic stabilization, interspinous spacer and/or adjacent level fusion devices). The artificial caudad joint is configured to present a surface ***429*** that receives the surface 427 of the cephalad bearing_***426***. Thus, for example, forming mating convex/concave surfaces to facilitate movement of each component of the facet joint element ***420*.** The artificial caudad joint is further adapted to engage the base ***416*** of the fixation element ***412*** such that the artificial caudad joint can be adjusted during and/or after implantation of the fixation element and then locked in place using a base anchor ***418**.* The outwardly facing surface of the base anchor ***418*** can be adapted to engage, for example, a driver, such as a flathead screwdriver, a Phillips head screwdriver, or a hexalobe driver. The base anchor ***418*** is further adapted to secure the artificial caudad joint ***428*** to the base ***416*** of the adjustable connector ***414*.** While the fixation element ***412*** of the polyaxial connector ***414*** is depicted as incorporating threads to secure it to the vertebral body, it should be understood that a host of other fixation mechanisms, including textured/bony in-growth surfaces, expanding anchors, clamps and/or adhesives can be used.

The relative locations and/or orientations for the fixation elements ***412*** and ***422*** of the artificial facet joint ***410*** are generally mandated by the patient's natural anatomy (such as the locations, orientations and/or conditions of the pedicles, lamina, spinous process and/or vertebral bodies themselves), as well as the objectives of the surgical procedure, and the tissues and structures removed and/or modified during the surgical procedure. Desirably, these fixation elements will be optimally placed for secure fixation. However, optimal placement for secure fixation may not always equate to optimal placement for proper function of the various components of the facet replacement device, and thus there may be a need to adjust and/or alter the position(s) and/or orientation(s) of the artificial cephalad and caudad joints ***426*** and ***428*** relative to their respective fixation elements (which desirably can be accommodated by the previously-described adjustability of the caudad and cephalad components). Accordingly, after implantation and adjustment, the artificial caudad joint ***428*** and the artificial cephalad joint ***426*** may be in an anatomically correct position within the patient's body (relative to the anatomical structures they augment and/or replace) or in an non-anatomically correct position, depending on the desired clinical outcome and the condition of the spinal anatomy (including, e.g., whether the vertebra have been anatomically altered, either surgically or naturally), as well as any other considerations and requirements of the situation.

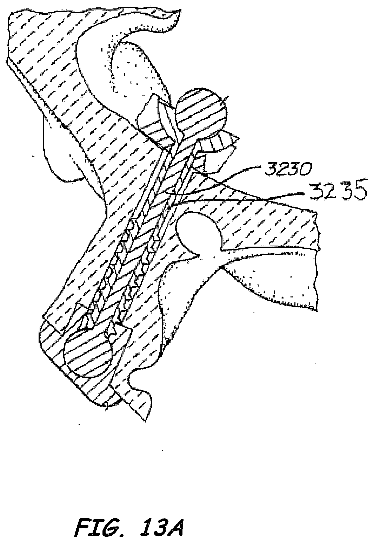

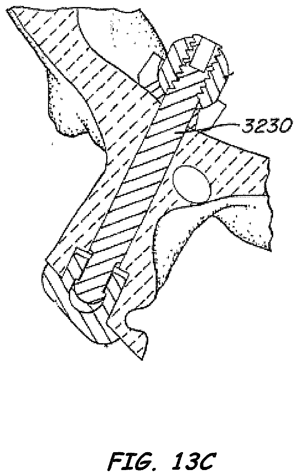

In one alternate embodiment, the fixation element could comprise a cable ***3230*** and cannulated tube ***3235*** (see **FIG. 13A**), with the cannulated tube passing through the targeted bony structure of the lamina and/or spinous process and encircling the cable along at least a portion of its length. The cable ***3230*** would desirably facilitate flexibility of the implanted artificial facet joint ***3100,*** while the cannulated tube would, among other things, significantly increase the cross-sectional surface area of the cable, thereby reducing the opportunity of the cable to "pull-out" of the bone laterally. A bearing engages one end of the cable and an anchor engages the opposing end. Alternatively, the fixation element could comprise a solid rod having
flexible or adjustable engagement member ***3250*** (see **FIG. 13C**) allowing for some variation between the orientation of the cephalad bearing and the longitudinal axis of the rod. In these embodiments, the cross-section of the cable/rod is desirably of a sufficient size to prevent the force applied by the cable in the lamina from exceeding the ability of the lamina to resist the force; *i.e*., a cable of insufficient diameter could present a force great enough to tear through the lamina. Moreover, the cross-section of the cable/rod need not be circular, but may be any shape, including irregular shapes, to desirably present a surface to the surrounding bone that is (1) large enough (and/or flat enough) to resist "pull-out" through the bone (e.g. along the length of the cable), (2) non-circular to resist rotation of the cable/rod, and/or (3) of increased surface area to facilitate bony in-growth into the cable/rod. The anchors can further be adapted to provide internal threads such that the anchors can be secured to the cable by screwing the anchor onto a threaded end of the cable, or can be adapted to provide an aperture that enables the anchor to be snap fit onto the end of the cable. Other configurations will be apparent to those skilled in the art.

The device ***410*** can be attached to a cut or resected portion of the vertebra. The cephalad portion of the device ***410*** is secured to the vertebral via an extension cable ***422*** that passes through the lamina (*see, **20*** of **FIG. 2A**) while the caudad portion of the device is secured to the vertebra by, for example, a pedicle anchor. As will be appreciated by those skilled in the art, the bearing surfaces ***427*, *429*** may be flat, spherical or a range of concavities/convexities and could be used in varying combinations. The bone-interfacing surface of the device can be configured to consist of any of a variety of surfaces that prevent relative motion and/or promote bone in-growth. Additionally, a pocket, or recess, can be provided to deliver substances to stimulate local bone growth (for example, BMPs, bone graft, bone graft substitutes, etc.) as well as to serve as a relative motion limiter once bone growth into the recess or pocket has occurred. Moreover, the cable ***422*** enables the device to achieve optimal angular and depth flexion. Further choosing the length of the cable ***422*** can further optimize the performance of the device ***410*** after implantation because the variable length of the cable can account for a wider variety of spinal anatomy. Further, the cable ***422*** can be tensioned as desired by the surgeon further adapting the device to a particular physiological condition to be corrected.

A portion of the vertebra may be surgically removed or altered, for example, to permit access to and removal of the superior facet of the caudad vertebra. A pedicle anchor ***412*** is then deployed and the caudad bearing is affixed to the anchor. Adjustability of the implanted device ***410*** may be achieved through the use of varying sized components as well as altering configuration and fit (e.g., with the use of polyaxial anchors, tapers, interlocking splines, etc.). Implantation of the device can also be accomplished using minimally invasive surgical techniques. Repair, replacement and/or augmentation can also be performed using limited-open, modified-open, and/or fully open surgical procedures. For example, where facet joint replacement is necessary, but removal of soft and/or hard tissue in and/or adjacent the spinal canal is not warranted or desired (such as where spinal stenosis and nerve inpingement is not a significant concern), the repair and/or replacement of one or more facet joints can be accomplished in a least-invasive fashion using one or more cannulae to implant the device and associated hardware. Alternatively, where the removal of the facet joint ***32*** and/or lamina ***20*** is necessitated (shown in **FIG. 2**), such a procedure can be accomplished through a combination of open, semi-open, and/or minimally invasive procedures to minimize damage and/or disruption to surrounding soft-tissue structures. In such a procedure, one or more of the facet joint capsules can be exposed through an open incision or semi-open procedure such as through an expanding cannula (to allow easy resection and removal of the facet joint, allow easy introduction of larger components of the facet joint, and/or surrounding anatomical structures), and the cephalad component of the facet replacement can be delivered through the lamina through a cannula or other minimally-invasive delivery method.

Another advantage of the embodiment is that the device is positioned within the lamina with only limited portions of the implant extending outwards from the vertebral body. This arrangement presents a low-profile to the surrounding soft tissue structure, resulting in less interaction between the device and the surrounding soft tissues, as well as less displacement of natural tissues due to the presence of the implant. Moreover, anchoring the cephalad portion of the device within the lamina and/or spinous process reduces and/or eliminates the opportunity for unwanted contact between the dura and the implanted device. Desirably, once the facet replacement components are implanted, the device will induce a healing response in the patient's body, causing formation of a capsule or pseudo-capsule of soft tissue (including scar tissue) around the articulating elements of the facet replacement prosthesis.

Further, based on the position of the caudad bearing surface, a translaminar aperture or hole can be placed through a percutaneous cephalad approach targeting the bearing surface. Through a caudad approach (which may be similarly minimally-invasive, limited open, open or through an expanding cannula), the cephalad bearing portion of the device is placed in a desired position/orientation and the tension cable ***422*** is drawn in a retrograde fashion through the translaminar opening formed in the lamina or spinous process. The cable is then tightened and secured to the superior aspect of the lamina ***20*.** Once the cable ***422*** is secured and the superior aspect of the lamina, the cephalad portion of the device is secured against the cut surface of the inferior facet. Using such a minimally invasive surgical approach, the posterior structures, except for the facet and facet capsules, can be left undisturbed.

As will be appreciated upon reviewing the entire specification, the device ***410*** will also work in conjunction with a total disc replacement. Use with a total disc replacement provides a solution for the total disc replacement contraindication of facet degeneration. Implantation of a total disc replacement device prior to implanting the facet restoration device ***410*** opens the disc space, aiding in any decompression of the joint that may be necessary. The device may be used unilaterally or bilaterally, depending on the nature of and stage of disease, and can be used at multiple levels of the spine. Similarly, removal of some or all of the facet structures (and lamina, etc.) of the targeted vertebral level may permit the passage of one or more components of the artificial disc replacement (or nucleus replacement, or annular repair material, and their respective tools) through the removed facet tissues via a lateral, posterior-lateral and/or posterior approach. The functions of the removed tissues can then be replaced by implanting the facet replacement prosthesis as described herein.

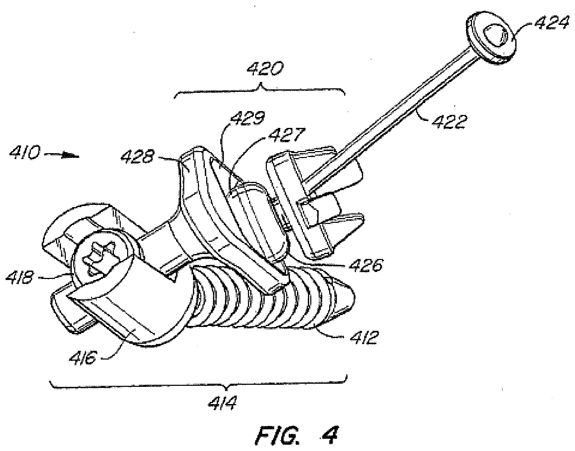

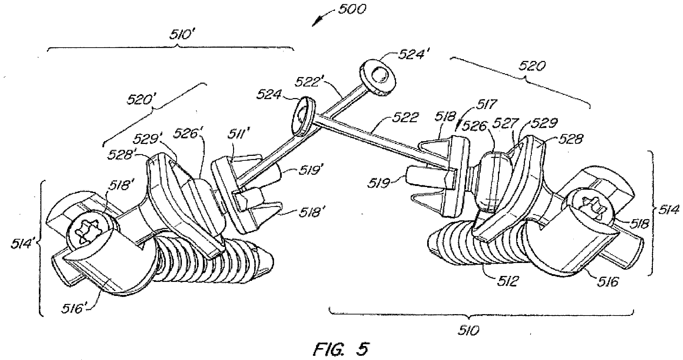

**FIG. 5** illustrates a bilateral facet replacement system ***500*** similar to the facet replacement device of **FIG. 4** illustrated as a bilateral solution (e.g., treating a facet joint on either side of the midline of the spine). The device is an artificial facet joint replacement device ***510**,* ***510*'** configured to replace a portion of a natural facet joint and a fixation element ***512, 512'*** that enables the device ***510, 510*'** to connect to a facet joint via, for example, an adjustable connection ***514, 514'**.* The connection ***514, 514*'** permits artificial caudad joints ***528, 528' 520, 520'*** and caudad bases ***516, 516'*** to be adjusted with respect to the fixation elements ***512, 512'*** about more than one axis. As discussed above, the construction can be adapted and configured to permit continuous adjustment through relative rotation of the facet joint element and the fixation element around many different axes through an adjustability range, up to a motion limit provided by a limit stop. In other embodiments, however, the number of axes of rotation may be limited, and the movement may be permitted only in discrete increments, In various embodiments the facet joint element may be moved medially, laterally, superiorly and/or inferiorly with respect to the fixation element. The facet j oint element ***520, 520'*** is further comprised of a flexible rod or cable ***522, 522',*** which is securable through, for example, the lamina and/or spinous process by use of an anchor ***524, 524'**.* The cable ***522*, *522*'** is adapted to engage an artificial cephalad joint ***526, 526'** having* a surface ***527, 527'*** adapted to mate with an opposing surface of an artificial caudad joint ***528, 528'**.* The artificial caudad joint is configured to present a surface ***529, 529'*** that received the artificial cephalad joint. Thus, for example, forming mating convex/concave surfaces to facilitate movement of each of the cephalad and caudad components ***526, 528*** of the facet joint element ***520, 520'.*** The artificial caudad joint is further adapted to engage the caudad base ***516, 516'*** of the fixation element **512, *512'*** such that the artificial caudad joint can adjusted during implantation and then locked in place using a base anchor.

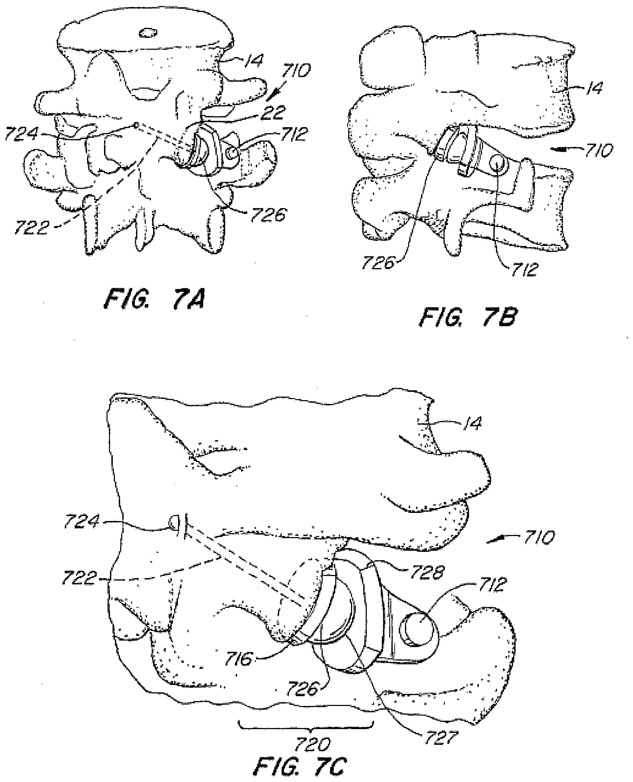

The relative positions of facet joint ***510, 510'*** and fixation element ***512, 512'*** may be set prior to implant, after implant, or both before and after implant. After implant and adjustment, the artificial caudad joint ***528, 528'*** and the artificial cephalad joint ***526, 526'*** may be in an anatomically correct position within the patient's body or in an non-anatomically correct position, depending on the desired clinical outcome and/or the condition of the spinal anatomy (*e.g*., whether the vertebra have been anatomically altered, either surgically or naturally), as well as any other considerations and requirements of the situation. In this embodiment, the cephalad component of the device is secured to the lamina (shown in **FIGS. 2A-D****, *20*)** of the cephalad vertebral body (*e.g., **14*** of **FIG. 2B****)** using flexible cables or rods ***522, 522'**.* The cables ***522, 522*'** can be configured to pass through some or all of the laminar surface, as will be better appreciated with respect to, for example, **FIG. 7****.** The inner surface ***511, 511*'** of the cephalad facet joint component ***526, 526'*** can be further adapted and configured to incorporate a textured, biologic or bony in-growth surface which promotes and/or allows biological in-growth, thereby augmenting attachment of the component of the surface to the vertebral body. Augmentation suitable for bony in-growth can be provided using, for example, resorbable bone cement, which increases the strength of the surface. The device can also be used in combination with bone filler or allograft material. Suitable bone filler material includes, the use of bone material derived from demineralized allogenic or xenogenic bone and can contain substances for example, bone morphogenic protein, which induce bone regeneration at a defect site. *See,* U.S. Patent Nos. 5,405,390 to O'Leary et al. for Osteogenic Composition and Implant Containing Same; 5,314,476 to Prewett et al. for Demineralized Bone Particles and Flowable Osteogenic Composition Containing Same; 5,284,655 to Bogdansky et al. for Swollen Demineralized Bone Particles, Flowable Osteogenic Composition Containing Same and Use of the Compositions in the Repair of Osseous Defects; 5,510,396 to Prewett et al. for Process for Producing Flowable Osteogenic Composition Containing Demineralized Bone Particles; 4,394,370 to Jeffries for Bone Graft Material for Osseous Defects and Method of Making Same; and 4,472,840 to Jeffries for Method of Inducing Osseous Formation by Implanting Bone Graft Material, which disclose compositions containing demineralized bone powder. *See also* U.S. Patent No. 6,340,477 to Anderson for Bone Matrix Composition and Methods for Making and Using Same, which discloses a bone matrix composition.

One or more prongs or projections ***518, 519*** are positioned on the inner surface of the base ***517*** of the cephalad facet joint component to prevent rotation and/or secure the component to the lamina. For example, the projections ***518*** are positioned such that they penetrate the laminar surface while the flattened projections ***519*** are positioned to desirably lay adjacent or against the outer surface of the lamina. As will be appreciated by those skilled in the art, the laminar surface can be prepared prior to the implanting the device, *e.g*., through resection of the articulating facet surface and/or the laminar surface, to provide a surface which, when abutting against the cephalad component will properly orient and position the cephalad component relative to an adjoining caudad bearing component. The cephalad facet joint component ***526*** has a bearing surface ***527*** that engages a bearing surface ***529*** of a caudad facet joint component ***528*.**

The components can be secured into and through the pedicles of the vertebral body. A multi-axial or polyaxial anchor can be used to permit in situ adjustment of the caudad bearing surface. Alternatively, an adjustable component can be used that permits adjustment. As will be appreciated by those skilled in the art, other anchors can be employed without departing from the scope of the invention.

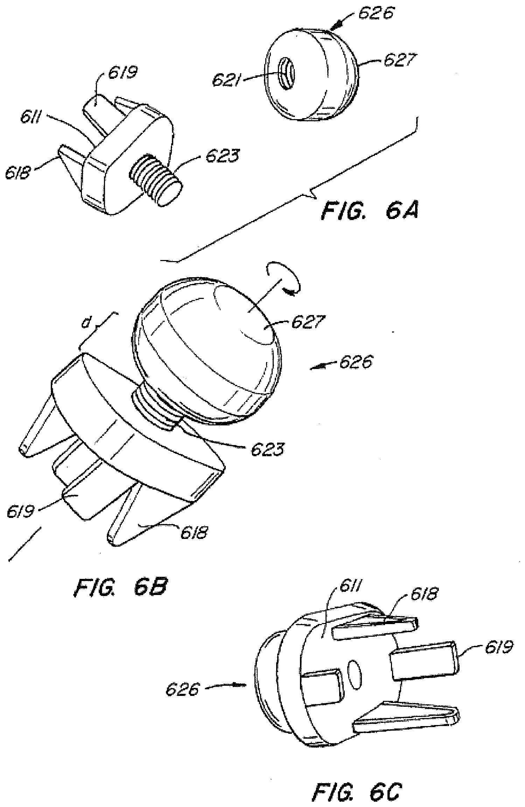

**FIG. 6A** illustrates two components of the cephalad facet joint portion of the facet replacement system shown in **FIGS. 4-5****.** One or more prongs or projections ***618, 619*** are positioned on the inner surface ***611*** of the cephalad facet joint component to prevent rotation and/or secure the component to the lamina of a vertebral body. The artificial cephalad joint component ***626*** has a bearing surface ***627*** that is adapted to enable the surface to engage a mating caudad joint surface. The artificial cephalad joint component ***626*** is configured to be removable from a base element by use of an aperture ***621*** on the joint component and threads ***623*** on the base component. The distance ***d*** between the upper surface of the joint component and the surface of the base component can be adjusted by controlling the rotating the joint element about the threads ***623*** on the base component. Thus, depending upon how far down onto the neck of the threaded base member the joint component is turned will effect the overall height of the device. Thus adaptability enables the device to be adjusted in situ to provide a better anatomical fit within a particular patient. **FIGS. 6B-C** illustrate the two components illustrated in **FIG. 6A** in combination from different perspectives. As an alternative, a series of artificial cephalad joint components ***626*** of differing shapes, sizes and/or orientations and/or lengths can be provided to accommodate different objectives, including alteration of bearing height/orientation relative to the fixation element, to accommodate different loading conditions due to other surgical treatments (i.e., artificial disc replacement of the same or other spinal level, annular repair, nucleus replacement, dynamic stabilization, interspinous spacer and/or adjacent level fusion and/or facet replacement devices). Moreover, to accommodate differing designs (i.e., constrained discs versus unconstrained discs) and/or arrangement/positioning of artificial disc replacement devices used on the same or different spinal levels, the artificial cephalad joint components ***626*** (and their respective artificial caudad joint components) could be of differing shapes, sizes, orientations and/or lengths to accommodate the different loading profiles induced or desired by the artificial disc replacement devices.

**FIGS. 7A-C** depict a comparative facet replacement device, similar to those depicted in
**FIGS**. **4-6****,** implanted within a vertebral body ***14*.** As shown in **FIG. 7****,** during implantation, the cephalad facet surface can be prepared and then the proximal end of the flexible cable or rod ***722*** is secured to the vertebral body. As can be seen from **FIG. 7****,** the device ***710*** as implanted is configured to replace a portion of a natural facet joint. The fixation element enables the device ***710*** to connect to a facet joint The facet joint element ***720*** is further comprised of a flexible rod or cable ***722*,** which is securable through, for example, the lamina and/or spinous process ***22*** by use of an anchor ***724*.** The cable ***722*** is adapted to engage an artificial cephalad joint ***726*** having a surface ***727*** adapted to mate with an opposing surface of an artificial caudad joint ***728*.** During a surgical implantation, the cephalad facet surface of the vertebral body can be prepared prior to implantation of the device. Thereafter, the proximal end of the flexible cable or rod is inserted into and through the lamina/spinous process in a desired direction and orientation with a first end of the cable ***722*** attached to the artificial cephalad joint component 726 and the second end adapted to engage an anchor ***724*** or cable lock. The anchor, or other securing device (not shown), can be attached (*e.g*., threaded) onto the end of the cable ***722*** and abutted against the lamina ***20*** of the vertebral body ***14*.** A tensioning tool and/or crimper, or similar device, can be used to tension the cable, thereby drawing the projections of the caudad base ***716*** toward the laminar surface. As will be appreciated by those skilled in the art, as the base is drawn toward the laminar surface, the projections (shown in **FIGS. 4-6****)** may be drawn into and/or against the lamina, depending upon the actual configuration of the projections. Thereafter, once a desired tension has been reached, the device can then be secured by locking the cable with the anchoring device ***724*.** Excess cable can be resected, if desired. As described above with respect to **FIG. 6****,** the cephalad component (as well as the respective caudad component) can be configured to provide an adjustable and/or removable bearing surface. Thus, the bearing surface location can be tailored to achieve the performance needs for the facet replacement device for a particular patient.

The caudad component ***728*** can also be secured into and through the pedicles of the caudad vertebral body. As illustrated, the caudad component is secured to the vertebral body by use of a fixation element ***712*.**

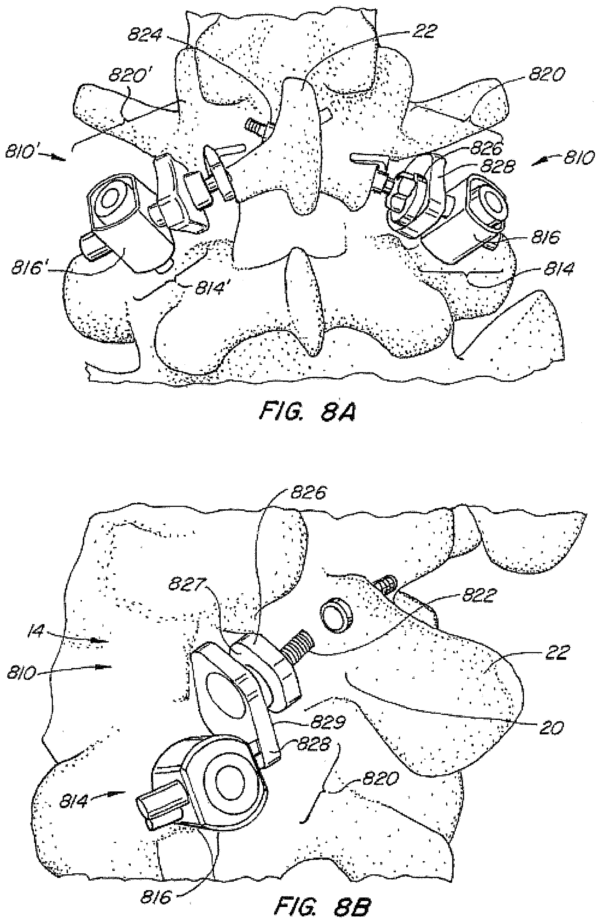

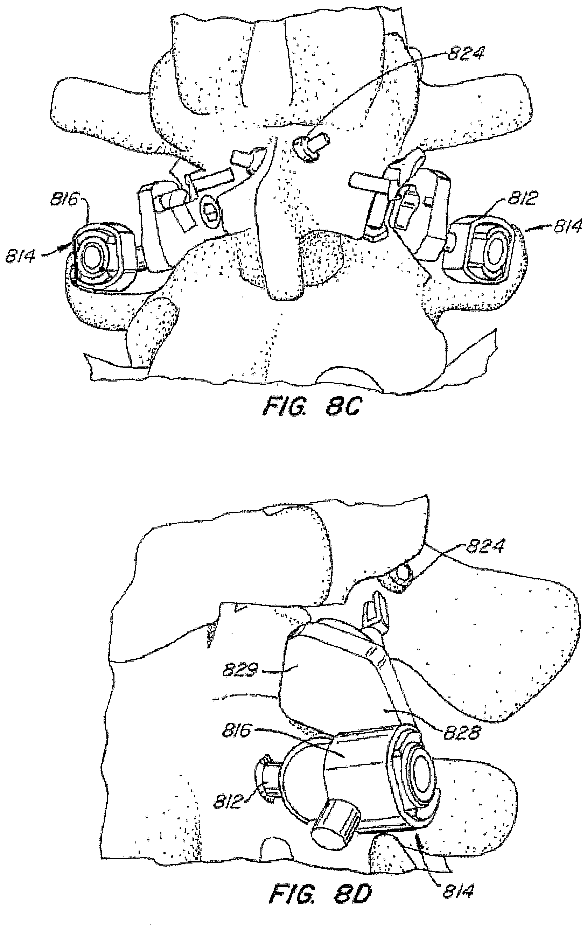

**FIGS. 8A-D** illustrate an implanted facet replacement device according to another embodiment of the invention. The device ***810*** as implanted is configured to replace a portion of a natural facet joint. The fixation element enables the device ***810*** to connect to a facet joint via, for example, a polyaxial connection ***814*.** The connection ***814*** permits the artificial caudad joint ***828*** and caudad base ***816*** to be adjusted with respect to the fixation element ***812*** around more than one axis within the patient. As can be appreciated by reviewing **FIG. 8**, the facet joint element may be moved medially, laterally, superiorly and/or inferiorly with respect to the fixation element attached to the vertebral body. The facet joint element ***820*** is further comprised of an anchoring stem ***822,*** which is securable through, for example, the lamina and/or spinous process ***22*** by use of a lock ***824.*** The anchoring stem ***822*** is adapted to engage an artificial cephalad joint ***826*** having a surface ***827*** adapted to mate with an opposing surface ***829*** of an artificial caudad joint ***828***. During a surgical implantation, the cephalad facet surface of the vertebral body can be prepared prior to implantation of the device. Thereafter, the proximal end of the flexible cable or rod is inserted into and through the lamina/spinous process in a desired direction and orientation with a first end of the anchoring stem ***822*** attached to the artificial cephalad joint component ***826*** and the second end adapted to engage an anchor ***824*** or cable lock. The anchor, or other securing device, can be attached (*e.g*., threaded) onto the end of the anchoring device ***822*** and abutted against the lamina ***20*** of the vertebral body ***14***. A tensioning tool and/or crimper, or similar device, can be used to provide sufficient torque to lock the anchor ***824*** onto the anchoring stem ***822***, thereby drawing the projections of the cephalad base ***817*** toward the laminar surface. As discussed above, as the base is drawn toward the laminar surface, the projections may be drawn into and/or against the lamina, depending upon the actual configuration of the projections. Thereafter, once a desired tension has been reached, the device can then be secured by locking the anchoring stem ***822*** with the anchoring device ***824*.** As described above with respect to **FIG. 6****,** the cephalad component (as well as the respective caudad bearing surface) can be configured to provide an adjustable and/or removable bearing surface. Thus, the bearing surface(s) can be tailored to achieve the performance needs for the facet replacement device for a particular patient. As further described above, the caudad component in this embodiment is configured to include a multi-axial or poly-axial component that is adjustable to permit *in situ* adjustment of the caudad bearing surface.

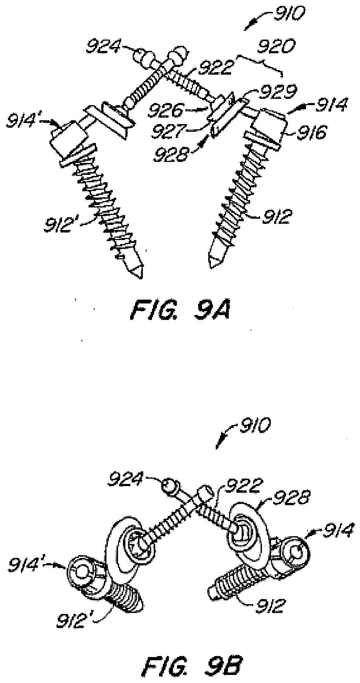

**FIGS. 9A-B** illustrate a facet replacement device according to another embodiment of the invention from a side view and a top view. The device ***910*** can be implanted to replace a portion of a natural facet joint. The fixation element ***912*** enables the device ***910*** to connect to a facet joint via, for example, an adjustable (*e.g.*, polyaxial) connection ***914.*** The connection ***914*** permits artificial caudad joint ***928*** and caudad base ***916*** to be rotated with respect to the fixation element ***912*** around more than one axis within the patient, if desired. As can be appreciated by reviewing **FIG. 9****,** the facet joint element may be moved medially, laterally, superiorly and/or inferiorly with respect to the fixation element attached to the vertebral body. The facet joint element ***920*** is further comprised of a cephalad threaded anchoring stem ***922,*** which is securable through, for example, the lamina and/or spinous process ***22*** and secured at a proximal end by a lock ***924*.** The anchoring stem ***922*** is adapted to engage an artificial cephalad joint ***926*** having a surface ***927*** adapted to mate with an opposing surface ***929*** of an artificial caudad joint ***928.*** The caudad joint ***928*** is configured to engage the threaded anchoring stem. Thus, when the anchoring stem is secured to the vertebra, the position of the caudad joint surface ***929*** can be adjusted to optimize the position of the caudad joint relative to the cephalad joint.

As with previous embodiments, during a surgical implantation, the cephalad facet surface of the vertebral body can be prepared prior to implantation of the device. Thereafter, the proximal end of the cable or rod (which is flexible) is inserted into and through the lamina and/or spinous process in a desired direction and orientation with a first end of the anchoring stem ***922*** attached to the artificial cephalad joint component ***926*** and the second end adapted to engage an anchor ***924*** or cable lock. The anchor, or other securing device, can be attached (*e.g*., threaded) onto the end of the anchoring device ***922*** and abutted against the bony surface or lamina ***20*** of the vertebral body ***14*.** A tensioning tool and/or crimper, or similar device, can be used to provide sufficient torque to lock the anchor ***924*** onto the anchoring stem ***922*** (if desired), thereby drawing the projections of the base ***916*** toward the laminar surface. As discussed above, as the base is drawn toward the laminar surface, the projections may be drawn into and/or against the lamina, depending upon the actual configuration of the projections. Thereafter, once a desired tension has been reached, the device can then be secured by locking the anchoring stem ***922*** with the anchoring device ***924*.** Desirably, this arrangement will allow much of the loading experienced by the artificial cephalad joint component ***926*** to be compressive in nature, thereby allowing transfer of a significant amount of these forces directly into the laminar surface, rather than to the cable or rod ***922*.** As described above with respect to **FIG. 6****,** the cephalad component can be configured to provide an adjustable and/or removable bearing surface. Thus, the bearing surface location can be tailored to achieve the performance needs for the facet replacement device for a particular patient. As further described above, the caudad component in this embodiment is configured to include an adjustable, multi-axial or poly-axial component that is adjustable to permit *in situ* adjustment of the caudad bearing surface.

**FIGS**. **10A-C** illustrate the facet replacement device of **FIGS.9A-B** implanted from a posterior and lateral perspective. In this embodiment, the caudad attachment is notched on the facet surface. The notching enables the device to engage a prepared or resected surface when implanted.

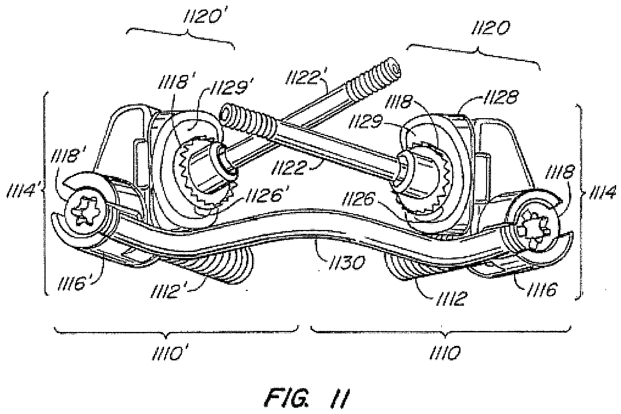

Turning now to, **FIG. 11** a bilateral facet replacement system with a caudad cross-bar is illustrated. The device includes a pair of artificial facet joint replacement devices ***1110, 1110'*** configured to replace a portion of a natural facet joint on either side of a vertebral body, which includes fixation elements ***1112, 1112*** that enable the devices ***1110, 1110'*** to connect to a caudad vertebral body via, for example, a polyaxial connector ***1114, 1114'.*** A cross-bar ***1130*** is provided connecting each of the caudad components of the artificial facet joint replacement devices ***1110, 1110'*** at their respective adjustable connectors ***1114, 1114'***. The cross-bar is formed of a bar having a plurality of curves enabling it to avoid disrupting portions of the spinal anatomy when implanted. Implantation of the cross-bar can be accomplished in a minimally-disruptive fashion by making a small vertical incision in the interspinous ligaments on the targeted spinal motion segment, and then threading the cross-bar through the opening created therein. Desirably, the cross-bar will prevent rotation of the caudad components and permits load sharing between their respective anchors.

As discussed above, the construction can be adapted and configured to permit continuous adjustment through relative rotation of the facet joint element and the fixation element around many different axes through an adjustability range. In other embodiments, however, the number of axes of rotation may be limited, and the movement may be permitted only in discrete increments. In various embodiments the facet joint element may be moved medially, laterally, superiorly and/or inferiorly with respect to the fixation element. The facet joint elements ***1120, 1120'*** are further comprised of an anchoring stem ***1122, 1122',*** which is securable through, for example, the lamina and/or spinous process. The anchoring stems ***1122, 1122'*** are adapted to engage an artificial cephalad joint ***1126,1126'*** having a surface adapted to mate with an opposing surface of the artificial caudad joint ***1128, 1128'.*** The artificial caudad joint is configured to present a surface that received the artificial cephalad joint. Thus, for example, forming mating convex/concave bearing surfaces enables the movement of each of the respective cephalad and caudad components ***1126, 1128*** of the facet joint element ***1120*** to occur more smoothly. The artificial caudad joint is further adapted to engage the base ***1116, 1116'*** of the fixation element ***1112, 112'*** such that the artificial caudad joint can adjusted during implantation and then locked in place using a base anchor.

The relative positions of each of the facet joints ***1110, 1110'*** and fixation elements ***1112, 1112'*** may be set prior to implant, after implant, or both before and after implant. After implant and adjustment, the artificial caudad joint ***1128, 1128'*** and the artificial cephalad joint ***1126, 1126'*** may be in an anatomically correct position within the patient's body or in an non-anatomically correct position, depending on the desired clinical outcome and/or the condition of the spinal anatomy (*e.g*., whether the vertebra have been anatomically altered, either surgically or naturally), as well as any other considerations and requirements of the situation. In this embodiment, the cephalad component of the device is secured to the lamina (shown in **FIGS. 2A**-**D**, ***20*)** of the cephalad vertebral body (*e.g., **14*** of **FlG**. **2B**) using an anchoring stem ***1122, 1122'.*** The anchoring stem can be provided with threads ***1109, 1109'*** that facilitate engaging the bone or adapted to engage an anchor at its end. As will be appreciated by those skilled in the art, the anchoring stem used in this embodiment can be a solid rod, or can be a cable, such as that depicted in **FIG. 5****,** which is configured to pass through some or all of the laminar surface. The cross-bar ***1130*** is adapted to traverse the midline of the spine when the device is implanted and to engage the base ***1116, 1116'*** of the connector ***1114, 1114'**.*

The facet replacement components can be further adapted to incorporate artificial ligaments between the articulating arms and/or the treated vertebral bodies. Alternatively, the devices could incorporate a flexible capsule around some or all of the facet/articulating joint surfaces. As will be appreciated by those skilled in the art will appreciate, multiple attachment points can be included, *e.g*., with the use of apertures, holes, hooks, etc. For attaching existing ligaments, tendons and/or other soft or hard tissues at the conclusions of the surgical procedure to promote healing and further stabilization of the affected level. Moreover, the natural healing response of the body may create a pseudo-capsule of soft and/or scar tissue abound the cephalad and caudad articulating surfaces of the facet replacement device, which may in some manner serve to duplicate some of the functions of the natural facet capsule.

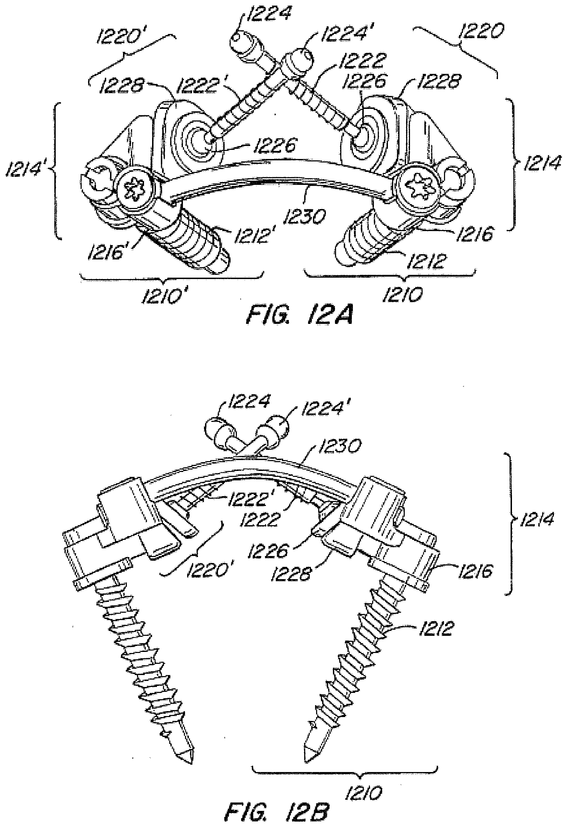

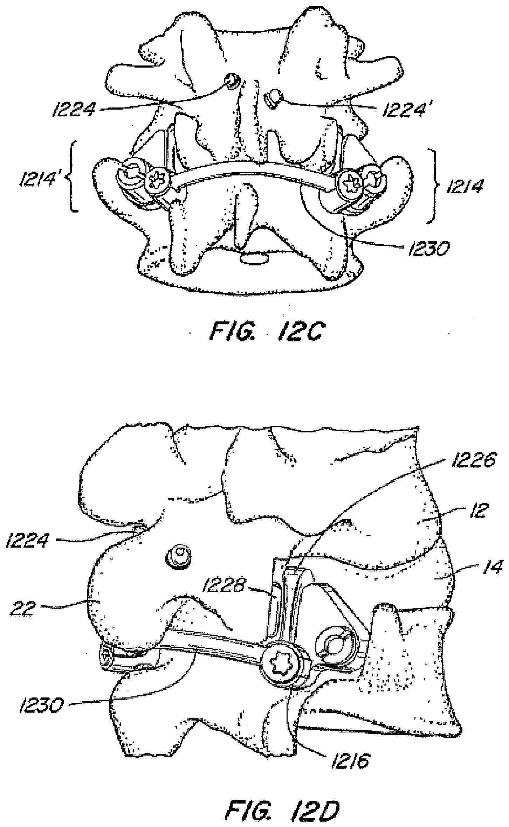

**FIGS. 12A-B****,** a bilateral facet replacement system with a cross-bar is depicted. Similar to the embodiment shown in **FIG. 11**, the device includes a pair of artificial facet joint replacement devices ***1210***, ***1210***' configured to replace a portion of a natural facet joint on either side of a vertebral body, including a fixation element ***1212***, ***1212*** that enables the devices ***1210, 1210'*** to connect to a caudad vertebral body via, for example, an adjustable connector ***1214,1214'.*** A cross-bar ***1230*** is provided connecting each of the artificial facet joint replacement devices ***1210, 1210'*** at their respective adjustable connectors ***1214, 1214'.*** The cross-bar can be formed of a curved bar that is adapted to follow the exterior curve of the vertebral body. More than one adjustable connector can be provided on each side of the device to enhance the ability of the device to accommodate a wide variety of anatomical differences upon implantation.

Similar to other embodiments, the connector ***1214, 1214'*** permits the artificial caudad joint ***1228, 1228'*** and caudad base ***1216, 1216'*** to be moved and/or rotated with respect to the fixation element ***1212, 1212'*** The facet joint elements ***1220, 1220'*** further incorporate anchoring stems ***1222, 1222'*** which are securable through, for example, the lamina and/or spinous process. The anchoring stems ***1222, 1222'*** are adapted to engage a artificial cephalad joint ***1226***, ***1226***' having a surface ***1227, 1227'*** adapted to mate with an opposing surface of the artificial caudad joint ***1228***, ***1228***'. The artificial caudad joint is configured to present a surface ***1229, 1229*** that received the artificial cephalad joint.

As with other embodiments, the relative positions of each of the facet joints ***1210, 1210'*** and fixation elements ***1212, 1212'*** may be set prior to implant, after implant, or both before and after implant. After implant and adjustment, the artificial caudad joint ***1228, 1228'*** and the artificial cephalad joint ***1226, 1226'*** may be in an anatomically correct position within the patient's body or in an non-anatomically correct position, depending on the desired clinical outcome and/or the condition of the spinal anatomy, as well as any other considerations and requirements of the situation. **FIGS. 12C-D** illustrates the facet replacement system implanted from the posterior and lateral perspectives.

In some embodiments it may be desirable to incorporate artificial ligaments between the articulating arms and/or the treated vertebral bodies. Additionally, in some embodiments it could be desireable to incorporate a flexible capsule around some or all of the facet/articulating joint or its surfaces. Alternatively, the facet replacement device can be adapted to incorporate multiple attachment points (apertures, holes, hooks, etc.) for attachment of existing ligaments, tendons and/or other soft or hard tissues at the conclusion of the surgical procedure to promote healing and further stabilization of the affected levels.

The devices and components disclosed herein can be formed of a variety of materials, as would be known in the art. For example, where the devices have bearing surfaces (*i.e*. surfaces that contact another surface), the surfaces may be formed from biocompatible metals such as cobalt chromium steel, surgical steel, titanium, titanium alloys (such as Nitinol), tantalum, tantalum alloys, aluminum, *etc.* Suitable ceramics, including pyrolytic carbon, and other suitable biocompatible materials known in the art can also be used. Suitable polymers include polyesters, aromatic esters such as polyalkylene terephthalates, polyamides, polyalkenes, poly(vinyl) fluoride, PTFE, polyarylethyl ketone, and other materials that would be known to those of skill in the art. Various alternative embodiments of the spinal devices and/or components could comprise a flexible polymer section (such as a biocompatible polymer) that is rigidly or semi rigidly fixed such that the polymer flexes or articulates to allow the vertebral bodies to articulate relative to one another.

Various embodiments of the present invention relate to a total spine joint replacement system comprising a modular facet joint replacement in combination with an artificial spinal disc replacement device. Virtually all of the various embodiments disclosed here could be utilized, in various ways, in combination with artificial disc replacement devices, as well as nucleus repair systems and replacement devices, interbody spacers, dynamic stabilization devices, articulating rod and screw systems, posterior ligament or annular repair and/or augmentation devices, interspinous spacers, facet resurfacing devices, and the like, with varying utility.

Various embodiments of the present invention desirably link the facet replacement prosthesis with the artificial disc replacement prosthesis in some manner. This link can be integral, such that the two components are "hard linked" together (either inflexibly, or flexibly- to allow and/or disallow articulation between components), or the components can be "soft linked" together, to allow movement and/or displacement between the components to some desired limit. If desired, at least one end of the linking device can comprise a polyaxial-type connection to connect to one or components of the facet replacement prosthesis. In alternate embodiments, the link may similarly pass through one or more openings formed through the various facet replacement components.

Desirably, the limitations and disadvantages inherent with many prior art facet replacement systems, as well as many artificial disc replacement systems, can be reduced, minimized and/or eliminated by the combination of such systems into a single, functional spinal unit joint replacement system. For example, the opportunity for the disc replacement to migrate and/or displace relative to the vertebral bodies during the life of the implantation may be reduced and/or eliminated by linking the disc replacement to the facet replacement prosthesis. Similarly, linking the disc replacement to the facet replacement may confer the added benefit of reducing (or redistributing) loading of the anchors (pedicle, lamina, spinous process and/or some combination thereof) of the facet replacement prosthesis, or visa versa (attachment of the disc replacement to the facet replacement affects loading of the disc replacement). Moreover, the forces acting on one component of the device (i.e., the artificial disc replacement device) may be balanced and/or negated by various forces acting on another component of the device (i.e., the facet joint replacement device), thus reducing and/or balancing the forces acting on the entire construct and/or its anchoring devices.

In one embodiment, the connection mechanism between the linkage and the artificial disc replacement can further serve to augment the stability and long-term viability of the artificial disc replacement. In this embodiment, the linkage comprises a longitudinally-extending arm which travels along the endplate of the vertebral body, through an opening formed in the artificial disc replacement, and extending further along the endplate. Desirably, this arm will serve to distribute loading of the disc on the endplate, reducing and/or eliminating subsidence of the disc replacement into and/or through the vertebral endplate (in a manner similar to using a rescue ladder on thin ice to distribute the weight of the rescuer). Various embodiments of the arm can comprise a flattened or half-circular cross-section, with the flattened section (towards the endplate) comprising a bioactive and/or in-growth surface to promote biofixation to the surrounding tissues. The linkage arms could comprise flexible or rigid materials.

In one alternate embodiment, the linkage arms are desirably non-parallel and/or non symmetric between the upper and lower linkage arms (which are linked to the upper and lower components of the disc replacement, respectively), so as to provide both lateral and anterior/posterior support to prevent migration of the disc replacement device and/or more easily allow controlled displacement of the disc replacement upon manipulation of the linkage arms.

If desired, a displaceable/repositionable disc replacement system (as described in the paragraph above) could incorporate one or more "settings" that would allow the physician to control, limit, reduce, increase or prevent motion of the disc replacement and/or facet replacement devices (to promote some clinical benefit, including inducing spinal fusion, limit articulation to promote healing of spinal tissues, limit or allow micro motion to promote bony in-growth into devices, or some other desired clinical outcome).

In various embodiments, the linkage between the facet replacement prosthesis and the disc replacement device facilitates positioning (or repositioning) of the respective prosthesis/device relative to each other, to more easily allow matching (or compatibility) of the kinematics and/or performance characteristics of the prosthesis/devices to each other (desirably, to emulate the natural spinal joint).

In various embodiments, the disc replacement device could incorporate openings or other docking features that could be utilized, at a later date (such as, for example, during a subsequent surgical procedure), to attach a facet replacement device (as disclosed herein) to the disc replacement. For example, where the disc replacement has been implanted, and the patient has healed from that surgery, but suffers spinal degeneration in the future (such as, for example, degenerated facets, spinal stenosis and/or spondylolitic slip of the treated spinal level), the level can be reopened, the facet replacement device attached to the existing disc replacement implant, and the surgical procedure completed. A similar arrangement could be contemplated for a facet replacement device that is initially implanted with openings or docking features that are later utilized during subsequent implantation of an artificial disk replacement prosthesis.

Various alternative embodiments of the present invention relate to laminar and/or pedicle based systems for replacing natural facets, the systems anchored to the vertebral bodies, with or without using cement and/or bony ingrowth surfaces to augment fixation.

As will be appreciated by those skilled in the art, the various embodiments disclosed herein can be adapted to account for location, length and orientation of, for example, the laminar passage created by the surgeon during implantation. The various embodiments can also be adapted to account for an individual patient's anatomical constraints. Thus, a limited number of component sizes and/or shapes can be configured from a kit to accommodate a large variety of anatomical variations possible in a patient. For example, a kit including a cephalad implant can include cephalad implants having various lengths from 20mm to 70mm, in, for example, 5 or 10 mm increments to accommodate passages/lamina having different lengths/thicknesses. Similarly the depth of apertures that accommodate a component can also be adapted to accommodate a patient.

Another advantage of various embodiments is that the use of the lamina and spinous process as an anchor point for the device enables the device to be implanted while avoiding the pedicles of the vertebral body. Alternatively, it may be desirous to utilize the pedicles of the vertebral body as an anchor point for the device while avoiding the lamina and spinous process. In various embodiments, the combination of translaminar and pedicular attachment (or a hybrid of both) may be most advantageous to the patient. For example, where facet replacement devices are implanted into multiple spinal levels, such as implantation of facet replacement devices across each of the L4-S1 levels, the use of a cephalad translaminar facet replacement device (in the L4 vertebra) in combination with a caudad pedicular-anchored facet replacement device (in the L5 vertebra) may be used in the L4-L5 level, while the use of a cephalad pedical-anchored facet replacement device (in the L5 vertebra - potentially utilizing the same pedicle anchors as for the caudad components of the L4-L5 level) in combination with a caudad pedicular-anchored device (in the sacrum) may be used in the L5-|S1 level. Such an arrangement would thus obviate the need to use the significantly weaker L5 lamina as an anchoring point, yet allow multiple level replacement of the facet joints. Such a hybrid device could, of course, similarly be used in conjunction with all manner of spinal treatment devices, including artificial disc replacements of one or more spinal levels, annular repair, nucleus replacement, dynamic stabilization, ligament repair and replacement, interspinous spacer, articulating rod and screw systems, and/or adjacent level fusion devices.

Additional disclosure useful in understanding the scope and teaching of the invention as it relates to intervertebral discs is in U.S. Patent Pubs. US 2005/0055096 A1 to Serhan et al., for Functional Spinal Unit Prosthetic; and US 2005/0033434 A1 to Berry for Posterior Elements Motion Restoring Device.

Further disclosures useful in understanding the scope and teaching of the invention are included in U.S. Patent No. 6,610,091, to Mark A. Reiley, for Facet Arthroplasty Devices and Methods; U.S. Publication Nos. US 2005/0283238 A1, to Mark A. Reiley, for Facet Arthroplasty Devices and Methods; US 2005/0234552 A1, to Mark A. Reiley, for Facet Arthroplasty Devices and Methods; US 2005/0267579 A1, to Mark A. Reiley, et al., for Implantable Device For Facet Joint Replacement; US 2006/0009849 A1, to Mark A. Reiley, for Facet Arthroplasty Devices and Methods; US 2006/0009848 A1, to Mark A. Reiley, for Facet Arthroplasty Devices and Methods; US 2006/0009847 A1, to Mark A. Reiley, for Facet Arthroplasty Devices and Methods; US 2004/0006391 A1, to Mark A. Reiley, for Facet Arthroplasty Devices and Methods; US 2004/0111154 A1, to Mark A. Reiley, for Facet Arthroplasty Devices and Methods; US 2004/0049276 A1, to Mark A. Reiley, for Facet Arthroplasty Devices and Methods; US 2005/0251256 A1, to Mark A. Reiley, for Facet Arthroplasty Devices and Methods;
US 2004/0049273 A1, to Mark A. Reiley, for Facet Arthroplasty Devices and Methods; US 2004/0049281 A1, to Mark A. Reiley, for Facet Arthroplasty Devices and Methods; US 2004/0049275 A1, to Mark A. Reiley, for Facet Arthroplasty Devices and Methods; U.S. Patent No. 6,949,123 B2, to Mark A. Reiley, for Facet Arthroplasty Devices and Methods; U.S. Publication Nos. US 2004/0049274 A1, to Mark A. Reiley, for Facet Arthroplasty Devices and Methods; US 2004/0049278 A1, to Mark A. Reiley, for Facet Arthroplasty Devices and Methods; US 2004/0049277 A1, to Mark A. Reiley, for Facet Arthroplasty Devices and Methods; US 2005/0137706 A1, to Mark A. Reiley, for Facet Arthroplasty Devices and Methods; US 2005/0137705 A1, to Mark A. Reiley, for Facet Arthroplasty Devices and Methods; US 2005/0149190 A1, to Mark A. Reiley, for Facet Arthroplasty Devices and Methods; US 2005/0043799 A1, to Mark A. Reiley, for Facet Arthroplasty Devices and Methods;
US 2002/0123806 A1, to Mark A. Reiley, for Facet Arthroplasty Devices and Methods; U.S. Patent No. 6,974,478, to Mark A. Reiley, et al., for Prostheses, Systems, and Methods for Replacement of Natural Facet Joints with Artificial Facet Joint Surfaces; US 2005/0240265 A1, to Mark Kuiper, et al., for Crossbar Spinal Prosthesis Having a Modular Design and Related Implantation Methods; US 2005/0119748 A1, to Mark A. Reiley, et al., for Prostheses, Systems, and Methods for Replacement of Natural Facet Joints with Artificial Facet Joint Surfaces;
US 2005/0027361 A1, to Mark A. Reiley for Facet Arthroplasty Devices and Methods; US 2005/0240266 A1, to Mark Kuiper, et al., for Crossbar Spinal Prosthesis Having a Modular Design and Related Implantation Methods; US 2005/0261770 A1, to Mark Kuiper, et al., for Crossbar Spinal Prosthesis Having a Modular Design and Related Implantation Methods; US 2004/0230201 A1, to Hansen Yuan, et al., for Prostheses, Systems, and Methods for Replacement of Natural Facet Joints with Artificial Facet Joint Surfaces; US 2005/0143818 A1, to Hansen Yuan, et al., for Prostheses, Systems, and Methods for Replacement of Natural Facet Joints with Artificial Facet Joint Surfaces; US 2005/0010291 A1, to David Stinson, et al., for Prostheses, Systems, and Methods for Replacement of Natural Facet Joints with Artificial Facet Joint Surfaces; U.S. Application No. 11/275,447 to David Stinson, et al., for Prostheses, Systems, and Methods for Replacement of Natural Facet Joints with Artificial Facet Joint Surfaces; US 2004/030304 A1**,** to Hansen Yuan, et al., for Prostheses, Systems, and Methods for Replacement of Natural Facet Joints with Artificial Facet Joint Surfaces; US 2005/0131406 A1, to Mark A. Reiley, et al., for Polyaxial Adjustment of Facet Joint Prostheses; US 2005/0240264A1, to Leonard Tokish, et al., for Anti-rotation Fixation Element for Spinal Prostheses; US 2005/0235508 A1, to Teena M. Augostino, et al., for Facet Joint Prostheses Measurement and Implant tools; U.S. Application No. 11/236,323, to Michael J. Funk, For Implantable Orthopedic Device Component Selection Instrument and Methods; U.S. Application No. 11/206,676, to Richard Broman, et al., for Implantable Spinal Device Revision System; US 2006/0041211 A1, to Teena M. Augostino, et al., for Adjacent Level Facet Arthroplasty Devices, Spine Stabilization Systems, and Methods; US 2006/0041311 A1, to Thomas J. McLeer for Devices and Methods for Treating Facet Joints; U.S. Application Nos. 11/140,570, to Thomas J. McLeer, for Methods and Devices for Improved Bonding to Bone; and 11/244,420, to Thomas J. McLeer, for Polymeric Joint Complex and Methods of Use.

While preferred embodiments of the present invention have been shown and described herein, it will be obvious to those skilled in the art that such embodiments are provided by way of example only.

## Claims

1. A facet joint restoration device (410, 510) for use in restoring a facet joint surface comprising:
(a) a cephalad facet joint element comprising: a flexible member (422, 522) adapted to engage a first vertebrae and an artificial cephalad joint (426, 526); and
(b) a caudad facet joint element comprising a connector having a screw or a stem (412, 512) that is adapted for fixation to a second vertebrae and an artificial caudad joint (428, 528) adapted to engage the artificial cephalad joint,
**characterized in that** the connector further comprises a base (416, 516), an adjustable connection (414, 514) for connecting the base to the artificial caudad joint allowing adjustment and rotation of the artificial caudad joint along and around one or more axes relative to the screw or stem during and after implantation of the screw or stem, and a base anchor (418) for locking the artificial caudad joint in place.

2. The facet joint restoration device according to claim 1, wherein the flexible member (422, 522) is adapted to engage a lamina of the first vertebrae.

3. The facet joint restoration device according to claim 1, wherein the cephalad facet joint further comprises a plate (424, 524) with an anchoring mechanism adapted to engage a lamina of the first vertebrae.

4. The facet joint restoration device of claim 3, wherein the anchoring mechanism includes anchoring mechanisms selected from the group consisting of teeth, ridges, nubs, serrations, granulations, a stem, a screw and a spike.

5. The facet joint restoration device of claim 2, wherein the cephalad facet joint element further comprises a second anchoring mechanism for securing the cephalad facet joint element to the first vertebrae.

6. The facet joint restoration device according to claim 2, wherein the connector is adapted for fixation to a pedicle of the second vertebrae.

7. The facet joint restoration device according to claim 5, wherein the second anchoring mechanism comprises a bony in-growth surface.

8. The facet joint restoration device according to claim 1, wherein the device (410, 510) replaces tissue removed from the facet joint.

9. The facet joint restoration device according to claim 1, wherein the device (410, 510) is adapted to restore or maintain motion or mobility for the facet joint.

10. The facet joint restoration device according to claim 1, wherein a surface (427, 429) of one of the cephalad facet joint element or caudad facet joint element is adapted to contour to an opposing mating surface.

11. The facet joint restoration device according to claim 1, wherein the artificial caudad joint (428) is a caudad cup having a concave surface.

12. The facet restoration device according to claim 1, wherein the flexible member (422, 522) is a flexible cable.

## Patentansprüche

1. Facettengelenk-Wiederherstellungsvorrichtung (410, 510) für den Gebrauch beim Wiederherstellen einer Facettengelenkoberfläche, Folgendes umfassend:
(a) ein nach kranial weisendes Facettengelenkelement, Folgendes umfassend: ein flexibles Element (422, 522), angepasst, einen ersten Wirbel und ein künstliches nach kranial weisendes Gelenk (426, 526) in Eingriff zu nehmen; und
(b) ein nach kaudal weisendes Facettengelenkelement, umfassend einen Verbinder mit einer Schraube oder einem Schaft (412, 512), angepasst zum Befestigen an einem zweiten Wirbel, und ein künstliches nach kaudal weisendes Gelenk (428, 528), angepasst, das künstliche nach kranial weisende Gelenk in Eingriff zu nehmen,
**dadurch gekennzeichnet, dass** der Verbinder ferner Folgendes umfasst: eine Basis (416, 516), eine einstellbare Verbindung (414, 514) zum Verbinden der Basis mit dem künstlichen nach kaudal weisenden Gelenk, welche ein Einstellen und Drehen des künstlichen nach kaudal weisenden Gelenks entlang einer oder mehrerer Achsen und um diese herum mit Bezug auf die Schraube oder den Schaft während und nach der Implantation der Schraube oder des Schafts ermöglicht, und eine Basisverankerung (418) zum Fixieren des künstlichen nach kaudal weisenden Gelenks an einer Stelle.

2. Facettengelenk-Wiederherstellungsvorrichtung nach Anspruch 1, wobei das flexible Element (422, 522) angepasst ist, eine Lamina des ersten Wirbels in Eingriff zu nehmen.

3. Facettengelenk-Wiederherstellungsvorrichtung nach Anspruch 1, wobei das nach kranial weisende Facettengelenk ferner eine Platte (424, 524) mit einem Verankerungsmechanismus umfasst, angepasst, eine Lamina des ersten Wirbels in Eingriff zu nehmen.

4. Facettengelenk-Wiederherstellungsvorrichtung nach Anspruch 3, wobei der Verankerungsmechanismus Verankerungsmechanismen enthält, die ausgewählt sind aus der Gruppe bestehend aus Zähnen, Furchen, Stiften, Kerben, Körnungen, einem Schaft, einer Schraube und einem Dorn.

5. Facettengelenk-Wiederherstellungsvorrichtung nach Anspruch 2, wobei das nach kranial weisende Facettengelenkelement ferner einen zweiten Verankerungsmechanismus zum Sichern des nach kranial weisenden Facettengelenkelements an dem ersten Wirbel umfasst.

6. Facettengelenk-Wiederherstellungsvorrichtung nach Anspruch 2, wobei der Verbinder zum Fixieren an einem Pedikel des zweiten Wirbels angepasst ist.

7. Facettengelenk-Wiederherstellungsvorrichtung nach Anspruch 5, wobei der zweite Verankerungsmechanismus eine knöcherne Einwuchsoberfläche umfasst.

8. Facettengelenk-Wiederherstellungsvorrichtung nach Anspruch 1, wobei die Vorrichtung (410, 510) von dem Facettengelenk entferntes Gewebe ersetzt.

9. Facettengelenk-Wiederherstellungsvorrichtung nach Anspruch 1, wobei die Vorrichtung (410, 510) angepasst ist, Bewegung oder Beweglichkeit des Facettengelenks wiederherzustellen oder zu erhalten.

10. Facettengelenk-Wiederherstellungsvorrichtung nach Anspruch 1, wobei eine Oberfläche (427, 429) eines des nach kranial weisenden Facettengelenkelements oder des nach kaudal weisenden Facettengelenkelements angepasst ist, der Kontur einer gegenüberliegenden sich anfügenden Oberfläche zu entsprechen.

11. Facettengelenk-Wiederherstellungsvorrichtung nach Anspruch 1, wobei das künstliche nach kaudal weisende Gelenk (428) eine kaudale Schale mit einer konkaven Oberfläche ist.

12. Facetten-Wiederherstellungsvorrichtung nach Anspruch 1, wobei das flexible Element (422, 522) ein flexibles Seil ist.

## Revendications

1. Dispositif de restauration d'articulation facettaire (410, 510) destiné à être utilisé pour restaurer une surface d'articulation facettaire comprenant :
(a) un élément d'articulation facettaire en direction céphalique comprenant : un élément flexible (422, 522) adapté pour s'engager dans une première vertèbre et une articulation artificielle en direction céphalique (426, 526) ; et
(b) un élément d'articulation facettaire en direction caudale comprenant un raccord ayant une vis ou une tige (412, 512) qui est adapté pour être fixé à une seconde vertèbre et une articulation artificielle en direction caudale (428, 528) adaptée pour s'engager dans l'articulation artificielle en direction céphalique,
**caractérisé en ce que** le raccord comprend en outre une base (416, 516), un raccordement ajustable (414, 514) pour raccorder la base à l'articulation artificielle en direction caudale permettant un réglage et une rotation de l'articulation artificielle en direction caudale selon, et autour de, un ou plusieurs axes par rapport à la vis ou la tige pendant et après une implantation de la vis ou la tige, et un ancrage de base (418) pour bloquer l'articulation artificielle en direction caudale en place.

2. Dispositif de restauration d'articulation facettaire selon la revendication 1, dans lequel l'élément flexible (422, 522) est adapté pour s'engager dans une lame de la première vertèbre.

3. Dispositif de restauration d'articulation facettaire selon la revendication 1, dans lequel l'articulation facettaire en direction céphalique comprend en outre une plaque (424, 524) avec un mécanisme d'ancrage adapté pour s'engager dans une lame de la première vertèbre.

4. Dispositif de restauration d'articulation facettaire selon la revendication 3, dans lequel le mécanisme d'ancrage comporte des mécanismes d'ancrage choisis dans le groupe constitué de dents, de nervures, de petites bosses, de dentelures, de granulations, d'une tige, d'une vis et d'une pointe.

5. Dispositif de restauration d'articulation facettaire selon la revendication 2, dans lequel l'élément d'articulation facettaire en direction céphalique comprend en outre un second mécanisme d'ancrage pour arrimer l'élément d'articulation facettaire en direction céphalique à la première vertèbre.

6. Dispositif de restauration d'articulation facettaire selon la revendication 2, dans lequel le raccord est adapté pour être fixé à un pédicule de la seconde vertèbre.

7. Dispositif de restauration d'articulation facettaire selon la revendication 5, dans lequel le second mécanisme d'ancrage comprend une surface de développement osseux.

8. Dispositif de restauration d'articulation facettaire selon la revendication 1, dans lequel le dispositif (410, 510) remplace un tissu enlevé de l'articulation facettaire.

9. Dispositif de restauration d'articulation facettaire selon la revendication 1, dans lequel le dispositif (410, 510) est adapté pour restaurer ou maintenir un mouvement ou une mobilité de l'articulation facettaire.

10. Dispositif de restauration d'articulation facettaire selon la revendication 1, dans lequel une surface (427, 429) de l'un parmi l'élément d'articulation facettaire en direction céphalique ou l'élément d'articulation facettaire en direction caudale est adaptée pour épouser la forme d'une surface d'appariement opposée.

11. Dispositif de restauration d'articulation facettaire selon la revendication 1, dans lequel l'articulation artificielle en direction caudale (428) est une cavité en direction caudale ayant une surface concave.

12. Dispositif de restauration d'articulation facettaire selon la revendication 1, dans lequel l'élément flexible (422, 522) est un câble flexible.
